# EUROPEAN PATENT APPLICATION

(11) **EP 4 361 170 A1**
(43) Date of publication of application: **01.05.2024**
(21) Application number: 22828540.9
(22) Date of filing: 24.06.2022
(51) Int. Cl.: C07K 14/165, A61K 39/215, A61P 31/14, A61P 37/04

(54) **ANTIBODY-INDUCING POLYPEPTIDE AND VACCINE**

(30) Priority: 24.06.2021 JP 2021104912; 14.06.2022 JP 2022095590
(71) Applicant: Watanabe, Yoshihiro, Kanazawa-shi, Ishikawa, 920-1192 (JP)
(72) Inventor: KAWANO, Mitsuhiro, Kanazawa-shi, Ishikawa 920-1192 (JP); HOSOKAWA, Natsuko, Kanazawa-shi, Ishikawa 920-1192 (JP); MIURA, Tomoyuki, Kyoto-shi, Kyoto 606-8507 (JP); WATANABE, Yoshihiro, Kanazawa-shi, Ishikawa 920-1192 (JP)
(74) Representative: Altmann Stößel Dick Patentanwälte PartG mbB
(86) International application number: PCT/JP2022/025330
(87) International publication number: WO 2022/270624

(57) **Abstract**

The invention provides an antibody-inducing polypeptide that is useful for treatment or prevention of SARS-CoV-2 infection in a subject and a vaccine that comprises such antibody-inducing polypeptide. The polypeptide is selected from the group of polypeptides (a) to (f) below and has antibody-inducing properties: (a) a polypeptide consisting of 7 or more continuous amino acids in a region of positions 336 to 361, 406 to 432, or 446 to 480 of SEQ ID NO: 1; (b) a polypeptide comprising an amino acid sequence having 80% or higher sequence identity to the amino acid sequence of any polypeptide as defined in (a); (c) a polypeptide comprising the polypeptide as defined in (a) or (b) as a partial sequence and comprising no region other than the partial sequence of SEQ ID NO: 1; (d) a polypeptide consisting of 10 or more continuous amino acids in a region of positions 1144 to 1161 or 1174 to 1202 of SEQ ID NO: 1; (e) a polypeptide comprising an amino acid sequence having 80% or higher sequence identity to the amino acid sequence of any polypeptide as defined in (d); and (f) a polypeptide comprising the polypeptide as defined in (d) or (e) as a partial sequence and comprising no region other than the partial sequence of SEQ ID NO: 1.

## Description

### Technical Field

The present invention relates to an antibody-inducing peptide and a vaccine for treatment or prevention of infection with SARS-CoV-2.

### Background Art

Coronaviruses are a group of RNA viruses of order Nidovirales, family Coronaviridae, and subfamily Orthocoronavirinae. Coronaviruses are enveloped viruses with a positive-sense, single-stranded RNA genome and helically symmetrical nucleocapsids and have characteristic forms with club-shaped protrusions on the capsid surface. Coronaviruses are large viruses among various types of RNA viruses, and the genome size thereof is approximately 26 to 32 kb.

Coronaviruses are known as RNA viruses that cause infectious diseases in mammals, birds, and the like. In particular, coronaviruses are known to cause mild to severe respiratory tract infections. In humans, coronaviruses are responsible for common colds in the case of mild infection (many viruses causing colds other than coronaviruses are known), and some coronaviruses causing SARS, MERS, and the like are known to cause more lethal symptoms. In particular, the novel coronavirus, SARS-CoV-2, found in China in 2019 has spread all over the world within a short period of time, and there were no available therapeutic agents or vaccines. Thus, many people were infected with and died of the novel coronavirus. At present, the number of infected people is increasing, and rapid development and popularization of vaccines and therapeutic agents for SARS-CoV-2 are awaited.

SARS-CoV-2 has the spike protein that binds to the host cell receptor on the virus surface. The spike protein is a trimeric glycoprotein, and each polypeptide chain is composed of the N-terminal domain (NTD), the receptor-binding domain (RBD), and the S2 domain. It has been found that the receptor-binding domain (RBD) comprises a "down conformation" and an "up conformation" and that the up conformation of RBD binds to the angiotensin converting enzyme II (ACE2) receptor on the host cell surface (Non-Patent Literature 1). Meanwhile, the S2 domain of the coronavirus is known to be associated with virus-to-host cell membrane fusion in the event of infection (Non-Patent Literature 2).

In addition to the mRNA vaccine comprising mRNA that encodes a most part of the spike protein (S antigen) introduced into carrier molecules, such as lipid nanoparticles, at present, an adenovirus expression vector, an S-antigen-expressing DNA vaccine, a recombinant protein, and the like comprising the S-antigen gene sequence introduced thereinto have been developed as vaccines to prevent SARS-CoV-2 infection.

### Citation List

### Non Patent Literature

Non Patent Literature 1: D. Wrapp et al., Science, Vol. 367, pp. 1260-1263, 2020
Non Patent Literature 2: A. C. Wallis et al., Proc. Natl. Acad. Sci., U.S.A., Vol. 114, pp. 11157-11162, 2017

### Summary of Invention

### Object to be Achieved

The mRNA vaccine is advantageous in that, for example, it is non-infectious, effective in terms of production cost, and relatively easy to produce; however, the mRNA vaccine is problematic in that, for example, stability of RNA molecules is low, strong side effects are likely to appear, translation and expression efficiency *in vivo* is likely to vary among individuals.

Meanwhile, a vaccine using the complete S antigen potentially has a risk of causing antibody species that could induce antibody-dependent infection enhancement (ADE). The presence of an antibody that induces ADE is actually observed at given frequency in serum samples obtained from elderly patients with severe COVID-19 infection. In recent years, epitopes that are recognized by the ADE-inducible antibodies were identified with the use of serum samples from COVID-19 patients. By removing such epitopes from the vaccine, a risk of AED may be avoided.

The present invention provides an antibody-inducing polypeptide that is useful for treatment or prevention of SARS-CoV-2 infection in a subject and a vaccine that comprises such polypeptide.

### Means for Achieving the Object

The present inventors had extensively examined the S antigen and, in particular, the entire RBD region and the entire HR1/2 regions of the S2 domain which are the responsible regions for SARS-CoV-2 infection. As a result, a plurality of novel neutralizing antibody inducing polypeptides (neutralizing antibody epitopes) were found, and it is found that such polypeptides would be useful for antibody induction for treatment or prevention of SARS-CoV-2 infection. Also, a plurality of novel antibody epitopes recognized in common by serum samples from COVID-19 patients were found, and it was found that polypeptides comprising such epitope sequences would be useful for antibody induction for treatment or prevention of SARS-CoV-2 infection. These findings have led to the completion of the present invention.

Specifically, the present disclosure provides the following inventions.
(1) A polypeptide selected from the group of polypeptides (a) to (f) below and having antibody-inducing properties:
   (a) a polypeptide consisting of 7 or more continuous amino acids in a region of positions 336 to 361, 406 to 432, or 446 to 480 of SEQ ID NO: 1;
   (b) a polypeptide comprising an amino acid sequence having 80% or higher sequence identity to the amino acid sequence of any polypeptide as defined in (a);
   (c) a polypeptide comprising the polypeptide as defined in (a) or (b) as a partial sequence and comprising no region other than the partial sequence of SEQ ID NO: 1;
   (d) a polypeptide consisting of 10 or more continuous amino acids in a region of positions 1144 to 1161 or 1174 to 1202 of SEQ ID NO: 1;
   (e) a polypeptide comprising an amino acid sequence having 80% or higher sequence identity to the amino acid sequence of any polypeptide as defined in (d); and
   (f) a polypeptide comprising the polypeptide as defined in (d) or (e) as a partial sequence and comprising no region other than the partial sequence of SEQ ID NO: 1.
(2) The polypeptide according to (1), which is selected from the group of polypeptides (a) to (c) below:
   (a) a polypeptide consisting of 7 or more continuous amino acids in a region of positions 336 to 361, 406 to 432, or 446 to 480 of SEQ ID NO: 1;
   (b) a polypeptide comprising an amino acid sequence having 80% or higher sequence identity to the amino acid sequence of any polypeptide as defined in (a); and
   (c) a polypeptide comprising the polypeptide as defined in (a) or (b) as a partial sequence and comprising no region other than the partial sequence of SEQ ID NO: 1.
(3) The polypeptide according to (1), which is selected from the group of polypeptides (d) to (f) below:
   (d) a polypeptide consisting of 10 or more continuous amino acids in a region of positions 1144 to 1161 or 1174 to 1202 of SEQ ID NO: 1;
   (e) a polypeptide comprising an amino acid sequence having 80% or higher sequence identity to the amino acid sequence of any polypeptide as defined in (d); and
   (f) a polypeptide comprising the polypeptide as defined in (d) or (e) as a partial sequence and comprising no region other than the partial sequence of SEQ ID NO: 1.
(4) A vaccine used for treatment or prevention of SARS-CoV-2 infection comprising at least one polypeptide selected from among the polypeptides as defined in (1).
(5) The vaccine according to (4), which comprises at least one polypeptide selected from among the polypeptides as defined in (2) and at least one polypeptide selected from among the polypeptides as defined in (3).
(6) The vaccine according to (4) or (5), which comprises a conjugate of a polypeptide bound to a carrier molecule.
(7) The vaccine according to any of (4) to (6), which comprises a conjugate of a plurality of polypeptides bound to a carrier molecule.
(8) The vaccine according to (6) or (7), wherein the conjugate has a circular structure comprising polypeptides crosslinked on a carrier molecule.
(9) The vaccine according to (7) or (8), which is a mucosal vaccine.
(10) The vaccine according to (9), which is a sublingual vaccine.
(11) An antibody-inducing composition comprising at least one polypeptide selected from among the polypeptides as defined in (1).
(12) The polypeptide according to (1), which is a polypeptide consisting of 7 or more continuous amino acids in the amino acid sequence as shown in any of SEQ ID NOs: 40 to 44.
(13) A method for treatment or prevention of infection with SARS-CoV-2 comprising a step of administering the vaccine according to any of (4) to (10) to a subject.

The description incorporates the contents disclosed by JP Patent Application Nos. 2021-104912 and 2022-095590, based on which the priority of the present application claims.

### Advantageous Effects of Invention

The present invention can provide an antibody-inducing polypeptide that is useful for prevention of SARS-CoV-2 infection in a subject and a vaccine that comprises such polypeptide.

### Brief Description of Drawings

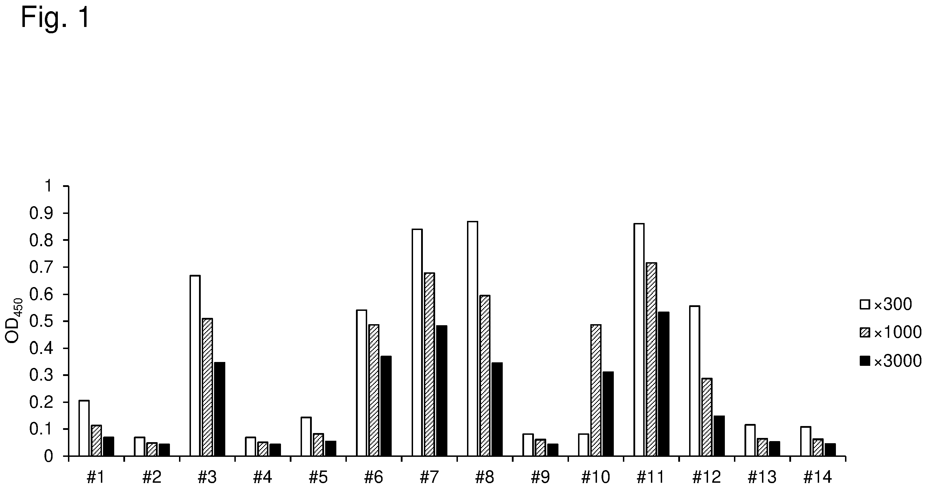

[Figure 1] Figure 1 shows a bar chart demonstrating the RBD protein-recognizing-ability of mouse serum IgG samples sensitized with various polypeptides of the SARS-CoV-2 RBD. The vertical axis indicates the binding ability of various serum IgG samples to the RBD protein.

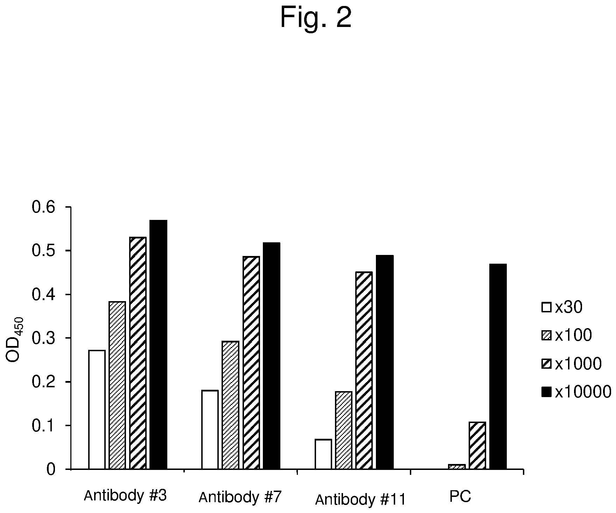

[Figure 2] Figure 2 shows a bar chart demonstrating the ability to inhibit the binding between ACE2 and RBD of mouse serum samples sensitized with polypeptides #3, #7, and #11 in SARS-CoV-2 RBD. The vertical axis indicates the binding-ability of ACE2 to RBD.

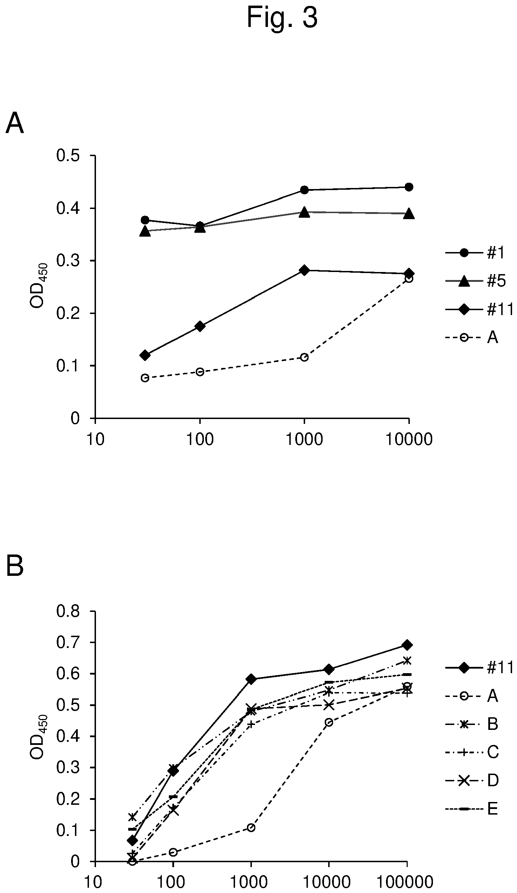

[Figure 3] Figure 3 shows charts demonstrating the ability to inhibit the binding between ACE2 and RBD of mouse serum samples sensitized with polypeptides #1, #5, and #11 in SARS-CoV-2 RBD and serum samples from patients recovered from SARS-CoV-2 infection. The vertical axis indicates the binding ability of ACE2 to RBD and the horizontal axis indicates dilution factors of serum samples.

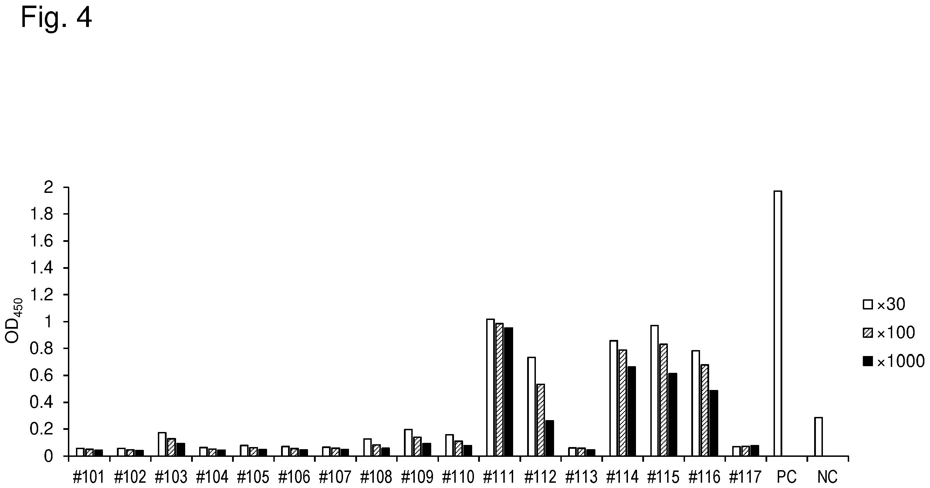

[Figure 4] Figure 4 shows a bar chart demonstrating the S2 domain protein-recognizing-ability of mouse serum IgG samples sensitized with various polypeptides of the SARS-CoV-2 S2 domain. The vertical axis indicates the binding ability of various serum IgG samples to the S2 domain protein.

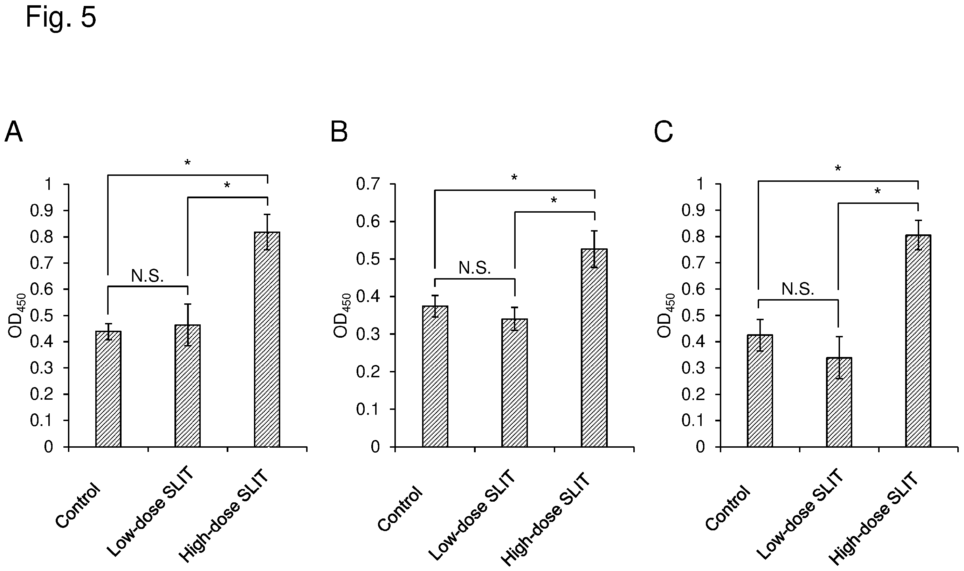

[Figure 5] Figure 5 shows bar charts demonstrating changes in IgG antibody levels caused by additional and repeated sublingual immunotherapy (SLIT) in mice sensitized with the mixture of conjugate polypeptides (#7, #11, #111, and #115). The vertical axis demonstrate the peptide recognizing ability of antibodies. (A) shows the reactivity of IgG in mouse serum samples of the high-dose SLIT group, the low-dose SLIT group, and the control group to the mixture of conjugates #7 and #11. (B) shows the reactivity of IgG in mouse serum samples of the high-dose SLIT group, the low-dose SLIT group, and the control group to the conjugate #111. (C) shows the reactivity of IgG in mouse serum samples of the high-dose SLIT group, the low-dose SLIT group, and the control group to the conjugate #115.

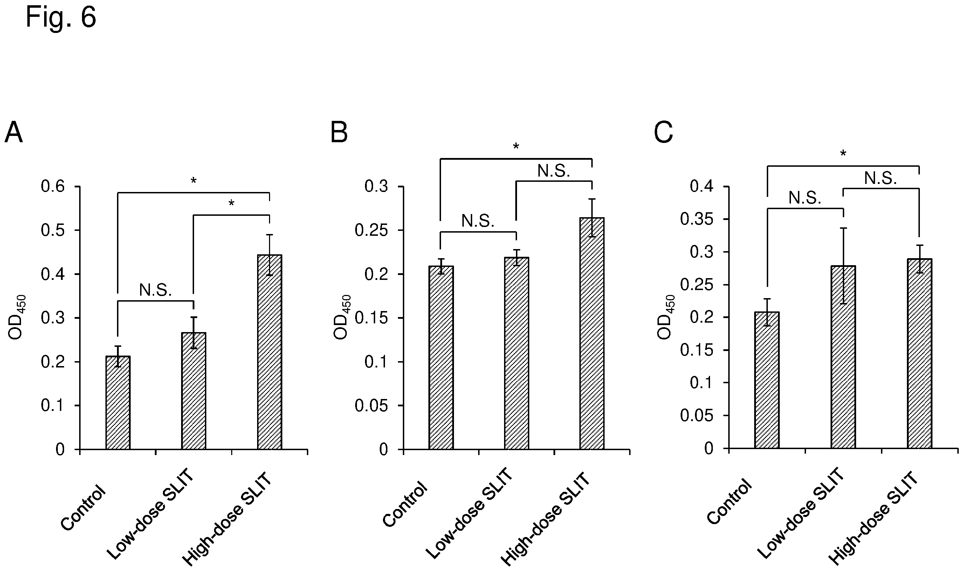

[Figure 6] Figure 6 shows bar charts demonstrating changes in IgA antibody levels caused by additional and repeated sublingual immunotherapy (SLIT) in mice sensitized with the mixture of conjugate polypeptides (#7, #11, #111, and #115). The vertical axis demonstrate the peptide recognizing ability of antibodies. (A) shows the reactivity of IgA in mouse serum samples of the high-dose SLIT group, the low-dose SLIT group, and the control group to the mixture of conjugates #7 and #11. (B) shows the reactivity of IgA in mouse serum samples of the high-dose SLIT group, the low-dose SLIT group, and the control group to the conjugate #111. (C) shows the reactivity of IgA in mouse serum samples of the high-dose SLIT group, the low-dose SLIT group, and the control group to the conjugate #115.

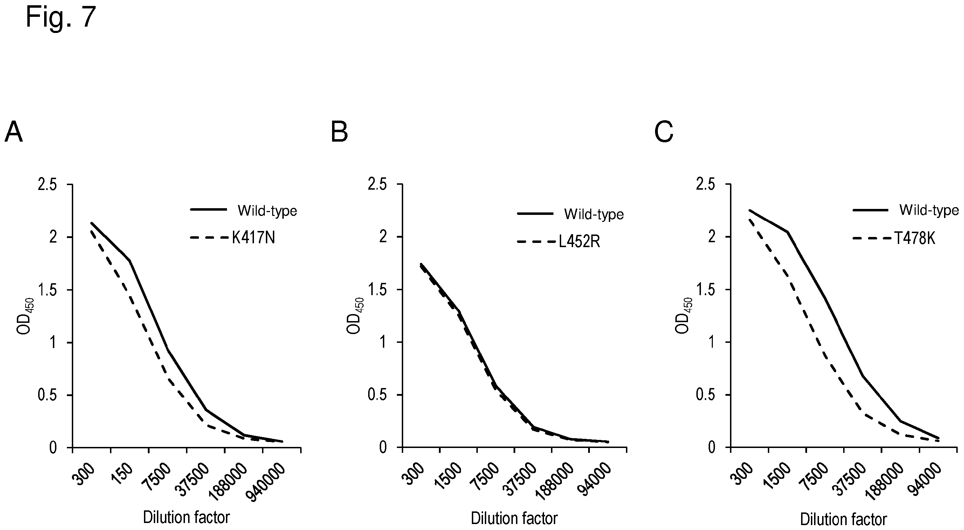

[Figure 7] Figure 7 shows charts demonstrating the reactivity of mouse serum samples sensitized with #7-, #10-, and #11-presenting antigens to the RBD epitope presenting antigen of mutant SARS-CoV-2. (A) shows the reactivity of the #7-recognizing serum to wild-type and K417N mutant RBD epitopes. (B) shows the reactivity of the #10-recognizing serum to wild-type and L452R mutant RBD epitopes. (C) shows the reactivity of the #11-recognizing serum to wild-type and T478K mutant RBD epitopes.

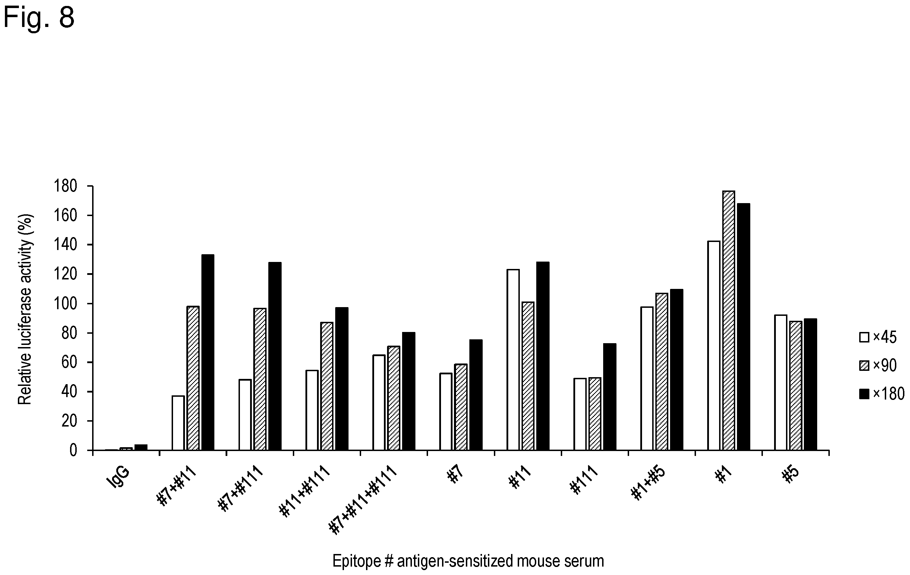

[Figure 8] Figure 8 shows a chart demonstrating the ability to protect ACE2-expressing CRFK cells from pseudotyped lentivirus infection of antiserum samples obtained from mice sensitized with various conjugate peptides and of a mixture of such serum samples.

### Description of Embodiments

### [1] Definition

The term "polypeptide" used herein refers to a molecule formed of a plurality of amino acids by peptide bonds. In addition to a polypeptide molecule formed of a large number of amino acids, a low-molecular-weight molecule with a small number of amino acids (oligopeptide) is within the scope of the polypeptide according to the present invention.

The term "antibody-inducing properties" used herein refers to properties of inducing humoral immunity to control SARS-CoV-2 infection in a subject and producing an antibody against SARS-CoV-2. In particular, the term refers to properties of inducing production of a neutralizing antibody against SARS-CoV-2 in a subject.

The term "vaccine" used herein refers to a pharmaceutical composition that is administered to induce immunity against the target pathogen (the antibody-producing ability) for the purpose of treatment or prevention of diseases or poor physical conditions of a subject caused by pathogens, such as bacteria, viruses, or allergens, that had invaded into the subject's body.

The term "subject" used herein refers to mammals and birds. Birds refer to animals that belong to Phylum Chordata, Subphylum Vertebrata, Class Aves, and examples thereof include chickens, domestic ducks, quails, gooses, wild ducks, turkeys, budgerigars, parrots, mandarin ducks, and swans. Mammals refer to animals that belong to Phylum Chordata, Subphylum Vertebrata, Mammalia, including humans and non-humans, and examples thereof include primates, such as humans and chimpanzees, pet animals, such as dogs and cats, livestock animals, such as cows, pigs, horses, sheep, and goats, rodents, such as mice and rats, and mammalian animals raised in zoos. The subject is preferably a human herein.

The "sequence identity" between amino acid sequences can be determined with the use of a protein search system, such as BLAST (https://blast.ncbi.nlm.nih.gov/Blast.cgi), with or without the introduction of gaps herein. The term "several" used herein refers to an integer of 2 to 10, and preferably an integer of 2 to 6, 2 to 4, 2 or 3, or 2.

The term "adjuvant" used herein refers to a substance that is administered to a subject together with an antibody-inducing substance in a vaccine to enhance the antibody inducing effects.

The term "compositing ratio" used herein is by weight, unless otherwise specified. The concentration indicated by "%" is by weight, unless otherwise specified.

### [2] Antibody-inducing polypeptide

The polypeptide according to the present invention is a polypeptide that is selected from the group of polypeptides (a) to (f) below and has antibody-inducing properties:
(a) a polypeptide consisting of 7 or more continuous amino acids in a region of positions 336 to 361, 406 to 432, or 446 to 480 of SEQ ID NO: 1;
(b) a polypeptide comprising an amino acid sequence having 80% or higher sequence identity to the amino acid sequence of any polypeptide as defined in (a);
(c) a polypeptide comprising the polypeptide as defined in (a) or (b) as a partial sequence and comprising no region other than the partial sequence of SEQ ID NO: 1;
(d) a polypeptide consisting of 7 or more continuous amino acids in a region of positions 1144 to 1161 or 1174 to 1202 of SEQ ID NO: 1;
(e) a polypeptide comprising an amino acid sequence having 80% or higher sequence identity to the amino acid sequence of any polypeptide as defined in (d); and
(f) a polypeptide comprising the polypeptide as defined in (d) or (e) as a partial sequence and comprising no region other than the partial sequence of SEQ ID NO: 1.

The polypeptide according to the present invention has antibody-inducing properties because of the constitution as described above. By administering such polypeptide in the form of, for example, a peptide vaccine or a conjugate vaccine to a subject, accordingly, the polypeptide can be useful as methods for preventing SARS-CoV-2 infection.

The spike protein associated with infection to a host cell with SARS-CoV-2 is known to have the amino acid sequence as shown in SEQ ID NO: 1. In particular, the receptor-binding domain (RBD) related to the binding to a host cell and the S2 domain with the membrane fusion to a host cell are responsible regions for SARS-CoV-2 infection. RBD is known to have the amino acid sequence as shown in SEQ ID NO: 2 (corresponding to amino acids 316 to 519 of the amino acid sequence as shown in SEQ ID NO: 1). The S2 domain is known to comprise the HR1 region and the HR2 region having the amino acid sequence as shown in SEQ ID NO: 3 (corresponding to amino acids 910 to 1032 of the amino acid sequence as shown in SEQ ID NO: 1) and the amino acid sequence as shown in SEQ ID NO: 4 (corresponding to amino acids 1141 to 1223 of the amino acid sequence as shown in SEQ ID NO: 1), respectively.

The present inventors discovered that, among polypeptides constituting the amino acid sequences of the RBD and the S2 domain of SARS-CoV-2, specific polypeptides would exert high antibody-inducing properties in mice and/or humans. The specific polypeptides comprise the sequences shown in SEQ ID NOs: 5 to 9 below.
CPFGEVFNATRFASVYAWNRKRISNC (SEQ ID NO: 5: 336-361)
EVRQIAPGQTGKIADYNYKLPDDFTGC (SEQ ID NO: 6: 406-432)
GGNYNYLYRLFRKSNLKPFERDISTEIYQAGSTPC (SEQ ID NO: 7: 446-480)
ELDSFKEELDKYFKNHTS (SEQ ID NO: 8: 1144-1161)
ASVVNIQKEIDRLNEVAKNLNESLIDLQE (SEQ ID NO: 9: 1174-1202)

The polypeptide of the present invention includes any of the polypeptides having the amino acid sequences as shown in SEQ ID NOs: 5 to 9, and the polypeptide of the present invention is not limited thereto. The polypeptide of the present invention includes a polypeptide having an amino acid sequence exhibiting 80% or higher, and preferably 85% or higher or 90% or higher sequence homology to the amino acid sequence as shown in any of SEQ ID NOs: 5 to 9. The polypeptide of the present invention also includes a polypeptide derived from any of the polypeptides having the amino acid sequences as shown in SEQ ID NOs: 5 to 9 by modification of one or several amino acids. In general, modification of the number of amino acids that is less than 20%, and, in particular, less than 15% or less than 10% of the total number of amino acids, or one or several amino acids in a given polypeptide is not considered to affect functions of the original polypeptide. In some embodiments, desirable functions of the original polypeptide may be enhanced. In fact, a modified polypeptide composed of an amino acid sequence derived from the original amino acid sequence by modification (i.e., substitution, deletion, addition, and/or insertion) of one or several amino acid residues is known to retain physiological activity of the original polypeptide (Mark et al., 1984, Proc. Natl. Acad. Sci., U.S.A., 81: 5662-5666, Zoller and Smith, 1982, Nucleic Acids Res., 10: 6487-6500, Dalbadie-McFarland et al., 1982, Proc. Natl. Acad. Sci., U.S.A., 79: 6409-6413). Accordingly, the polypeptides (b) and (e) above can exert antibody-inducing properties.

The polypeptide of the present invention includes the polypeptide comprising an amino acid sequence of continuous amino acids in any of SEQ ID NOs: 5 to 9 (including the modified polypeptide above) as a partial sequence wherein no other region of SEQ ID NO: 1 is comprised, in addition to the polypeptide having the continuous amino acids in any of SEQ ID NOs: 5 to 9; i.e., the polypeptides (c) and (f) above.

The 20 amino acids constituting a naturally occurring protein can be classified into groups depending on properties: neutral amino acids comprising low polar side chains (Gly, Ile, Val, Leu, Ala, Met, and Pro); neutral amino acids comprising hydrophilic side chains (Asn, Gln, Thr, Ser, Tyr, and Cys); acidic amino acids (Asp and Glu); basic amino acids (Arg, Lys, and His); and aromatic amino acids (Phe, Tyr, and Trp). Substitution in each of such groups is known to cause little changes in polypeptide properties. When amino acid residues in the amino acid sequences as shown in SEQ ID NOs: 5 to 9 are to be substituted, accordingly, substitution may be performed in each of such groups, so that antibody-inducing properties are more likely to be maintained.

A significant challenge for vaccine development is antibody-dependent enhancement (ADE). It is critical to overcome the problem of ADE concerning the antigen with high antibody-inducing properties. The ADE epitopes of SARS-CoV-2 were found to be regions shown in Table 1 in recent years (refer to doi.org/10.1101/2020.10.08.20209114). The polypeptide of the present invention is not any of the following ADE epitopes. Thus, the polypeptide of the present invention was found to have both high antibody-inducing properties and safety.

**[Table 1]**

| Positions in SEQ ID NO: 1 | Sequence |
|---|---|
| 304-323 | KSFTVEKGIYQTSNFRVQPT |
| 364-383 | DYSVLYNSASFSTFKCYGVS |
| 544-563 | NGLTGTGVLTESNKKFLPFQ |
| 564-583 | QFGRDIADTTDAVRDPQTLE |
| 574-593 | DAVRDPQTLEILDITPCSFG |

SARS-CoV-2 mutants, such as N501, E484, and L452, are reported, and the polypeptide of the present invention has the ability to inhibit infection of such major mutants.

A method for preparing the polypeptide of the present invention is not particularly limited, and any method for preparing a polypeptide known in the art can be employed. For example, a method of peptide solid phase synthesis, such as the method of Fmoc solid-phase synthesis or the method of Boc solid-phase synthesis, or polypeptide production with the aid of recombinant *E. coli* can be employed.

### [2-1] The polypeptide of the first aspect (the RBD polypeptide)

The polypeptide according to the first aspect of the present invention is selected from the group of polypeptides (a) to (c) below:
(a) a polypeptide consisting of 7 or more continuous amino acids in a region of positions 336 to 361, 406 to 432, or 446 to 480 of SEQ ID NO: 1;
(b) a polypeptide comprising an amino acid sequence having 80% or higher sequence identity to the amino acid sequence of any polypeptide as defined in (a); and
(c) a polypeptide comprising the polypeptide as defined in (a) or (b) as a partial sequence and comprising no region other than the partial sequence of SEQ ID NO: 1.

The polypeptide according to the first aspect of the present invention is in the RBD domain of SARS-CoV-2. The present inventors sensitized mice with, as conjugate vaccines, particular polypeptides having high antibody-inducing properties selected from among polypeptides in the RBD domain and found that the resulting antibody would inhibit the binding of SARS-CoV-2 RBD to the ACE2 receptor.

The particular polypeptides comprise the sequences shown in SEQ ID NOs: to 7.
CPFGEVFNATRFASVYAWNRKRISNC (SEQ ID NO: 5)
EVRQIAPGQTGKIADYNYKLPDDFTGC (SEQ ID NO: 6)
GGNYNYLYRLFRKSNLKPFERDISTEIYQAGSTPC (SEQ ID NO: 7)

The polypeptide according to the first aspect of the present invention includes the polypeptides having the sequences shown in SEQ ID NOs: 5 to 7. It should be noted that the polypeptide is not limited thereto and includes: (a) a polypeptide consisting of 7 or more continuous, and preferably 8, 9, 10, 11, 12, 13, 14, 15, 16, 17, 18, 19, 20, 21, or 22 or more continuous amino acids in a region of amino acids 336 to 361, 406 to 432 (preferably 411 to 427), or 446 to 480 (preferably 459 to 480) in SEQ ID NO: 1; (b) a polypeptide having an amino acid sequence exhibiting 80% or higher, and preferably 85% or higher, 90% or higher, or 95% or higher sequence identity to the amino acid sequence of any polypeptide as defined in (a); and (c) a polypeptide comprising the polypeptide as defined in (a) or (b) as a partial sequence and comprising no region other than the partial sequence of SEQ ID NO: 1. While many SARS-CoV-2 mutants are known, a polypeptide constituting RBD of any mutant SARS-CoV-2 may be used herein.

The polypeptide according to the first aspect of the present invention may be a polypeptide constituting RBD of a known mutant SARS-CoV-2 (mutant), such as a polypeptide consisting of 7 or more continuous amino acids in the amino acid sequence as shown in any of SEQ ID NOs: 40 to 44 below.
APGQTGNIADYNYKLPDDFTGC (SEQ ID NO: 40: K417N)
GGNYNYRYRLFRKSNLKPFERD (SEQ ID NO: 41: L452R)
SNLKPFERDISTEIYQAGNTPC (SEQ ID NO: 42: S477N)
SNLKPFERDISTEIYQAGSKPC (SEQ ID NO: 43: T478K)
SNLKPFERDISTEIYQAGNKPC (SEQ ID NO: 44: S477N/T478K)

When an antibody targeting a particular mutant is induced in a subject, use of a polypeptide comprising a corresponding amino acid sequence of the mutant is particularly preferable. Thus, a more effective antibody against a target mutant can be induced.

The polypeptide according to the first aspect of the present invention is administered to a subject, so that production of an antibody against RBD of SARS-CoV-2 can be induced in a subject. When SARS-CoV-2 invades into the body of a subject, accordingly, the binding of SARS-CoV-2 to the ACE2 receptor via RBD is inhibited, and infection can be thus prevented.

### [2-2] The polypeptide of the second aspect (the polypeptide of the S2 domain)

The polypeptide according to the second aspect of the present invention is selected from the group of polypeptides (d) to (f) below.
(d) a polypeptide consisting of 10 or more continuous amino acids in a region of positions 1144 to 1161 or 1174 to 1202 of SEQ ID NO: 1;
(e) a polypeptide having an amino acid sequence exhibiting 80% or higher sequence identity to the amino acid sequence of any polypeptide as defined in (d); and
(f) a polypeptide comprising the polypeptide as defined in (d) or (e) as a partial sequence and comprising no region other than the partial sequence of SEQ ID NO: 1.

The polypeptide according to the second aspect of the present invention constitutes the S2 domain of SARS-CoV-2. The present inventors identified particular polypeptides having high antibody-inducing properties from among polypeptides constituting the S2 domain.

The particular polypeptides comprise the sequences shown in SEQ ID NOs: 8 and 9.
ELDSFKEELDKYFKNHTS (SEQ ID NO: 8)
ASVVNIQKEIDRLNEVAKNLNESLIDLQE (SEQ ID NO: 9)

The polypeptide according to the second aspect of the present invention includes the polypeptides having the sequences shown in SEQ ID NOs: 8 and 9. It should be noted that the polypeptide is not limited thereto and includes: (d) a polypeptide consisting of 7 or more continuous, and preferably 8, 9, 10, 11, 12, 13, 14, 15, 16, 17, or 18 or more continuous amino acids in a region of amino acids 1144 to 1161 or 1174 to 1202 in SEQ ID NO: 1; (e) a polypeptide having an amino acid sequence exhibiting 80% or higher, and preferably 85% or higher or 90% or higher sequence identity to the amino acid sequence of any polypeptide as defined in (d); and (f) a polypeptide comprising the polypeptide as defined in (d) or (e) as a partial sequence. While many SARS-CoV-2 mutants are known, a polypeptide constituting the S2 domain of any mutant SARS-CoV-2 may be used herein.

The polypeptide according to the second aspect of the present invention is administered to a subject, so that production of an antibody against the S2 domain of SARS-CoV-2 can be induced in a subject. When SARS-CoV-2 invades into the body of a subject, accordingly, the membrane fusion of SARS-CoV-2 to a host cell via the S2 domain is inhibited, and infection can be prevented.

### [3] Vaccine

The vaccine according to the present invention is used for treatment or prevention of SARS-CoV-2 infection comprising at least one polypeptide selected from among the polypeptides of the present invention described in the "[2] Antibody-inducing polypeptide" section above. Specifically, the vaccine according to the present invention is a peptide vaccine against SARS-CoV-2.

The vaccine according to the present invention may consist of one polypeptide of the present invention or it may comprise a plurality of polypeptides of the present invention. The vaccine according to the present invention preferably comprises at least one polypeptide according to the first aspect and at least one polypeptide according to the second aspect described below.

### (i) The polypeptide of the first aspect

A polypeptide, which is selected from the group of polypeptides (a) to (c) below:
(a) a polypeptide consisting of 7 or more continuous amino acids in a region of positions 336 to 361, 406 to 432, or 446 to 480 of SEQ ID NO: 1;
(b) a polypeptide comprising an amino acid sequence having 80% or higher sequence identity to the amino acid sequence of any polypeptide as defined in (a); and
(c) a polypeptide comprising the polypeptide as defined in (a) or (b) as a partial sequence and comprising no region other than the partial sequence of SEQ ID NO: 1.

### (ii) The polypeptide of the second aspect

A polypeptide selected from the group of polypeptides (d) to (f) below:
(d) a polypeptide consisting of 10 or more continuous amino acids in a region of positions 1144 to 1161 or 1174 to 1202 of SEQ ID NO: 1;
(e) a polypeptide comprising an amino acid sequence having 80% or higher sequence identity to the amino acid sequence of any polypeptide as defined in (d); and
(f) a polypeptide comprising the polypeptide as defined in (d) or (e) as a partial sequence and comprising no region other than the partial sequence of SEQ ID NO: 1.

Specifically, the polypeptide of the first aspect is a polypeptide in the RBD domain of SARS-CoV-2, and the polypeptide of the second aspect is a polypeptide in the S2 domain of SARS-CoV-2. In a preferable embodiment, the vaccine of the present invention comprises a polypeptide in the RBD domain and a polypeptide in the S2 domain of SARS-CoV-2. When the vaccine comprising polypeptides in 2 domains is administered to a subject, the vaccine can inhibit both of the 2 steps of SARS-CoV-2 infection: a step of the binding to the ACE2 receptor; and a step of the membrane fusion to a host cell. Thus, a subject can be protected from SARS-CoV-2 infection in two aspects.

When the vaccine of the present invention comprises the polypeptide of the first aspect and the polypeptide of the second aspect, the compositing ratio thereof is not particularly limited. For example, the compositing ratio can be 1:10 to 10:1, preferably 1:5 to 5:1, and more preferably 1:1. When each of the polypeptides of the first aspect and the polypeptides of the second aspect comprises a plurality of polypeptides, the composition ratio of the total polypeptides of the first aspect and the total polypeptides of the second aspect is not particularly limited. For example, the compositing ratio can be 1:10 to 10:1, preferably 1:5 to 5:1, and more preferably 1:1.

The form of a polypeptide to be included in the vaccine of the present invention is not particularly limited, provided that antibody-inducing properties of the polypeptide are maintained. A polypeptide may be in the form of a linear peptide or a conjugate of a polypeptide bound to a carrier molecule. The term "carrier molecule" used herein refers to a molecule used to maintain or improve antibody-inducing properties of a polypeptide. Examples thereof include albumin, thyroglobulin, ovalbumin (OVA), dextran, Sepharose, and carrier molecules used in clinical settings, such as keyhole limpet hemocyanin (KLH) and CRM197. Use of OVA, KLH, and CRM197 is particularly preferable.

A binding form of a polypeptide in a conjugate is not particularly limited, provided that antibody-inducing properties of the polypeptide are maintained. A conjugate may comprise a single type of polypeptide bound to a carrier molecule, or a conjugate may comprise a plurality of types of polypeptides bound to a carrier molecule. When a plurality of types of polypeptides are bound to a carrier molecule, each polypeptide may be directly bound to a carrier molecule. A polypeptide may be linearly bound to a conjugate molecule, or polypeptides may be crosslinked to each other on carrier molecules via a linker structure to form a circular structure together with the carrier molecules. In particular, a closed circular conjugate is stable in the form of the epitope site, an original polypeptide conformation (e.g., a loop structure or a helix structure) is formed, and, accordingly, such closed circular conjugate is preferably used. As a linker structure, a heterofunctional linker reagent, such as succinimidyl 4-(N-maleimidomethyl)cyclohexane-1-carboxylate (SMCC), sulfo-SMCC, LC-SMCC, N-ε-malemidocaproyl-oxysuccinimide ester (EMCS), succinimidyl 4-(p-maleimidophenyl)butyrate (SMPB), or m-maleimidobenzoyl-N-hydroxysuccinimide ester (MBS), can be used.

A method for preparing the conjugate having a circular structure as described above is not particularly limited. For example, the conjugate can be prepared by the method described below. Two or more linker molecules (e.g., molecules having maleimide groups) are allowed to bind to a carrier molecule, such as OVA, polypeptides are added to the carrier molecule in the number that would not exceed the number of the linker molecules, and the reaction is then allowed to proceed. In the absence of an amino acid residue (e.g., cysteine) having an SH group at the polypeptide end, for example, a polypeptide may be prepared while being provided with cysteine residues (preferably 2 or 3 amino acid residues comprising cysteine residues) at the both ends in advance, and a structure comprising SH groups at the both ends can be prepared. The SH groups at the both ends bind to different maleimide molecules, the SH groups are crosslinked to each other, and a circular structure of a carrier molecule-a linker molecule-a polypeptide is thus formed.

The administration route of the vaccine of the present invention is not particularly limited, and the vaccine may be administered by any known method, such as oral, nasal, sublingual, subcutaneous, or intramuscular administration. In particular, a mucosal vaccine that is administered through, for example, an oral, nasal, or sublingual route is preferable. A sublingual vaccine is more preferable. The mucosal vaccine is known to induce the systemic immune responses represented by IgG or the like. In addition, the mucosal vaccine is known to efficiently induce the mucosal immunity on the mucosal surface where SARS-CoV-2 invades the body, and, in particular, antigen-specific secretory IgA responses. Thus, infection can be controlled in two aspects: i.e., prevention of SARS-CoV-2 invasion; and protection through the systemic immune response.

The vaccine of the present invention may comprise an adjuvant. Any adjuvant known in the art may be used. Examples of known adjuvants include the Freund's complete and/or incomplete adjuvants, vitamin E, Montanide, alum, poly IC and a derivative thereof (e.g., poly ICLC), squalene and/or tocophenol, QS-21 derived from Quillya saponaria saponin, MPL (SmithKline Beecham), QS21 (SmithKline Beecham), a lipopolysaccharide from Salmonella minnesota R595, the Salmonella, a non-toxic derivative thereof: i.e., 3-deacylated-4'-monophosphophoryl lipid (MPL) A, and QS-7, QS-17, QS-18, and QS-L1 (So H. S., et al., Molecular and cells, Vol. 7, pp. 178-186, 1997).

In the vaccine of the present invention, the compositing ratio of polypeptides (the total amount of various polypeptides) to adjuvants is not particularly limited. For example, a compositing ratio that is employed for common vaccines can be employed.

The vaccine of the present invention may comprise a pharmaceutically acceptable carrier, according to need, in addition to the antibody-inducing polypeptide. The term "pharmaceutically acceptable carrier" used herein refers to an additive that is generally used in the field of pharmaceutical technology. Examples thereof include an excipient, a binder, a disintegrator, a filler, an emulsifier, a flow regulating reagent, and a lubricant.

Examples of excipients include sugars, such as a monosaccharide, a disaccharide, cyclodextrin, and a polysaccharide (specific examples include, but are not limited to, glucose, sucrose, lactose, raffinose, mannitol, sorbitol, inositol, dextrin, maltdextrin, starch, and cellulose), metal salts (e.g., sodium chloride, sodium phosphate, calcium phosphate, calcium sulfate, magnesium sulfate, and calcium carbonate), citric acid, tartaric acid, glycine, low-, middle-, and high-molecular weight polyethylene glycol (PEG), a phospholipid, a lysophospholipid, cholesterol, fatty acid, Pluronic^{®}, kaolin, silicic acid, and combination of any thereof.

Examples of binders include a starch paste using corn, wheat, rice, or potato starch, a simple syrup, a glucose liquid, gelatin, Tragacanth, methylcellulose, hydroxypropyl methylcellulose, carboxymethylcellulose sodium, shellac, and polyvinylpyrrolidone.

Examples of disintegrators include the starch, lactose, carboxymethyl starch, crosslinked polyvinylpyrrolidone, agar, laminaran powder, sodium bicarbonate, calcium carbonate, alginic acid or sodium alginate, polyoxyethylene sorbitan fatty acid ester, sodium lauryl sulfate, monoglyceride stearate, and a salt of any thereof.

Examples of fillers include the sugar and/or calcium phosphate (e.g., tricalcium phosphate and calcium hydrogen phosphate).

Examples of emulsifiers include sorbitan fatty acid ester, glycerin fatty acid ester, sucrose fatty acid ester, and propylene glycol fatty acid ester.

Examples of flow regulators and lubricants include silicate, talc, stearate, and polyethylene glycol.

Such carriers are primarily used to facilitate formation of the dosage forms as described above and to maintain the dosage forms and pharmacological effects. Accordingly, adequate carriers may be used, according to need. In addition to the additives described above, the vaccine of the present invention can comprise, for example, a corrigent, a solubilizer, a suspension agent, a diluent, a surfactant, a stabilizer, an absorption promoter, an extender, a humectant, a moisturizing agent, an adsorbent, a disintegration inhibitor, a coating agent, a coloring agent, a preservative, an antioxidant, an aroma chemical, a flavoring agent, a sweetening agent, or a buffer, according to need.

The vaccine of the present invention can comprise other drugs, provided that pharmacological effects of the vaccine are maintained. In the case of an injection preparation, for example, the vaccine may comprise a given amount of antibiotics.

The dosage form of the vaccine of the present invention is not particularly limited, provided that effects of antibody-inducing polypeptides and other additional ingredients are maintained. An example of a dosage form for sublingual administration is a tablet. Examples of dosage forms for oral administration include a tablet, a pill, a capsule, granules, fine grains, powders, a syrup, an emulsion, and a suspension. Examples of dosage forms for subcutaneous administration include an injection preparation, a poultice, and a percutaneous absorption preparation. An example of a dosage form for intramuscular administration is an injection preparation.

It is preferable that the vaccine of the present invention comprise antibody-inducing polypeptides in an amount that is effective for treatment or prevention of SARS-CoV-2 infection and would not cause serious side effects. For example, it is preferable that the total amount of the antibody-inducing polypeptides (that does not comprise carrier molecules) to be administered be adjusted to 0.01 to 100 µg/kg of body weight, and, in particular, 0.1 to 5.0 µg/kg of body weight.

The administration frequency of the vaccine of the present invention to be administered is not particularly limited, provided that production of antibodies against SARS-CoV-2 is sufficiently induced and serious side effects would not be caused. For example, the vaccine can be administered once a day for a month. Alternatively, the vaccine can be administered intermittently at a frequency of once to three times a week for a maximum of a month. An administration period may be for 2 weeks, or, after drug withdrawal, sensitization may be performed by concentrated administration for 2 weeks prior to the epidemic season.

### [4] Antibody-inducing composition

The antibody-inducing composition of the present invention comprises at least one polypeptide selected from among the polypeptides of the present invention described in the "[2] Antibody-inducing polypeptide" section above.

The antibody-inducing composition of the present invention comprises at least one polypeptide selected from among the polypeptides of the present invention. When the antibody-inducing composition is administered to an animal, accordingly, an antibody against SARS-CoV-2 can be produced in the animal. A polyclonal antibody against SARS-CoV-2 can be prepared by obtaining a serum sample from the animal. A monoclonal antibody against SARS-CoV-2 can be prepared by establishing hybridomas from the spleen cells of the animal. The antibody-inducing composition of the present invention has a polypeptide with high antibody-inducing properties, and antibodies against SARS-CoV-2 can be produced efficiently.

The antibody-inducing composition of the present invention is administered to an animal, such as a rabbit, a guinea pig, a goat, a sheep, a cow, a pig, a rat, a mouse, a chicken, a camel, or an alpaca.

The antibody-inducing composition of the present invention may comprise only one or a plurality of polypeptides of the present invention. The antibody-inducing composition of the present invention may comprise at least one polypeptide selected from among the polypeptides according to the first aspect of the present invention and at least one polypeptide selected from among the polypeptides according to the second aspect of the present invention.

The form of a polypeptide to be comprised in the antibody-inducing composition of the present invention is not particularly limited, provided that antibody-inducing properties of the polypeptide is maintained. A polypeptide may be in the form of a linear peptide or a conjugate of a polypeptide bound to a carrier molecule. A binding form of a polypeptide in a conjugate is not particularly limited, provided that antibody-inducing properties of the polypeptide are maintained. A conjugate may comprise a single type of polypeptide bound to a carrier molecule, or a conjugate may comprise a plurality of types of polypeptides bound to a carrier molecule. When a plurality of types of polypeptides are bound to a carrier molecule, each polypeptide may be directly bound to a carrier molecule. A polypeptide may be linearly bound to a conjugate molecule, or polypeptides may be crosslinked to each other on carrier molecules via a linker structure to form a circular structure together with the carrier molecule.

The administration route of the antibody-inducing composition of the present invention is not particularly limited, and the antibody-inducing composition is generally administered via subcutaneous or intramuscular injection.

The antibody-inducing composition of the present invention may comprise an adjuvant. Any adjuvant known in the art may be used. In the vaccine of the present invention, the compositing ratio of polypeptides (the total amount of various polypeptides) to adjuvants is not particularly limited. For example, a compositing ratio that is employed for common vaccines can be employed.

It is preferable that the antibody-inducing composition of the present invention comprises antibody-inducing polypeptides in an amount at which antibodies are sufficiently produced and serious side effects are not caused in the animal to which the antibody-inducing composition is administered. For example, it is preferable that the total amount of the antibody-inducing polypeptides (that does not comprise carrier molecules) to be administered be adjusted to 0.01 to 100 µg/kg of body weight, and, in particular, 0.1 to 5.0 µg/kg of body weight.

Other detailed conditions, such as other ingredients to be included in the antibody-inducing composition of the present invention, are the same as those described in the " [3] Vaccine" section above, unless there is a particular discrepancy.

### [5] Method for treatment or prevention of SARS-CoV-2 infection

The method for treatment or prevention of SARS-CoV-2 infection according to the present invention (hereafter, it may be simply referred to as "the method for treatment according to the present invention") comprises a step of administering the vaccine of the present invention described in the "[3] Vaccine" section above to a subject.

The detailed conditions, such as the subject of the method for treatment according to the present invention, the dosage form of the vaccine to be administered, and the ingredients, are as described in the "[3] Vaccine" section above, unless there is a particular discrepancy.

### [Examples]

Hereafter, the present invention is described in greater detail with reference to the examples, although the technical scope of the present invention is not limited to the following examples.

### [Example 1] Preparation of conjugates of polypeptides having the SARS-CoV-2 partial sequence

The polypeptides #1 to #14 and #101 to #117 shown in Table 2 were prepared by Fmoc synthesis. The polypeptides without cysteine residues at the ends shown in Table 2 were processed with the addition of 1 to 3 amino acid residues, such as cysteine-serine-glutamine (CSG), to the N end and glutamine-serine-cysteine (GSC) to the C end. The purity and the molecular weight of the polypeptide were examined in accordance with conventional techniques by means of HPLC and mass spectrometry.

Subsequently, a circular conjugate of each polypeptide was prepared with the use of OVA as a carrier molecule. Imject^{™} maleimide-activated ovalbumin OVA (Thermo Fisher) was dissolved in a conjugation buffer in a tube, an aqueous solution of each polypeptide was added under ice cooling, and the resultant was then allowed to stand at room temperature overnight. After remaining maleimide groups were blocked with the addition of L-cysteine, the resultant was stored at -30°C.

**[Table 2]**

| Polypeptide # | Positions in SEQ ID NO: 1 | Sequence | Domain | SEQ ID NO: |
|---|---|---|---|---|
| #1 | 316-336 | SNFRVQPTESIVRFPNITNLC | RBD | 10 |
| #2 | 336-355 | CPFGEVFNATRFASVYAWNR | RBD | 11 |
| #3 | 343-361 | NATRFASVYAWNRKRISNC | RBD | 12 |
| #4 | 361-379 | CVADYSVLYNSASFSTFKC | RBD | 13 |
| #5 | 380-392 | YGVSPTKLNDLCF | RBD | 14 |
| #6 | 391-414 | CFTNVYADSFVIRGDEVRQIAPGQ | RBD | 15 |
| #7 | 411-432 | APGQTGKIADYNYKLPDDFTGC | RBD | 16 |
| #8 | 406-427 | EVRQIAPGQTGKIADYNYKLPD | RBD | 17 |
| #9 | 431-448 | GCVIAWNSNNLDSKVGGN | RBD | 18 |
| #10 | 446-467 | GGNYNYLYRLFRKSNLKPFERD | RBD | 19 |
| #11 | 459-480 | SNLKPFERDISTEIYQAGSTPC | RBD | 20 |
| #12 | 479-499 | PCNGVEGFNCYFPLQSYGFQP | RBD | 21 |
| #13 | 500-515 | TNGVGYQPYRVVVLSF | RBD | 22 |
| #14 | 504-525 | GYQPYRVVVLSFELLHAPATVC | RBD | 23 |
| #101 | 901-920 | QMAYRFNGIGVTQNVLYENQ | S2 HR1 | 24 |
| #102 | 911-929 | VTQNVLYENQKLIANQFNS | S2 HR1 | 25 |
| #103 | 920-940 | QKLIANQFNSAIGKIQDSLSS | S2 HR1 | 26 |
| #104 | 929-950 | SAIGKIQDSLSSTASALGKLQD | S2 HR1 | 27 |
| #105 | 943-964 | SALGKLQDWNQNAQALNTLVK | S2 HR1 | 28 |
| #107 | 963-985 | VKQLSSNFGAISSVLNDILSRLD | S2 HR1 | 29 |
| #108 | 976-995 | VLNDILSRLDKVEAEVQIDR | S2 HR1 | 30 |
| #109 | 988-1006 | EAEVQIDRLITGRLQSLQT | S2 HR1 | 31 |
| #110 | 999-1019 | GRLQSLQTYVTQQLIRAAEIR | S2 HR1 | 32 |
| #111 | 1144-1161 | ELDSFKEELDKYFKNHTS | S2 HR2 | 33 |
| #112 | 1154-1168 | KYFKNHTSPDVDLGD | S2 HR2 | 34 |
| #113 | 1163-1181 | DVDLGDISGINASVVNIQK | S2 HR2 | 35 |
| #114 | 1174-1193 | ASWNIQKEIDRLNEVAKNL | S2 HR2 | 36 |
| #115 | 1184-1202 | DRLNEVAKNLNESLIDLQE | S2 HR2 | 37 |
| #116 | 1197-1213 | LIDLQELGKYEQYIKWP | S2 HR2 | 38 |
| #117 | 1205-1223 | KYEQYIKWPWYIWLGFIAG | S2 HR2 | 39 |

### [Example 2] Sensitization of mice with conjugates

Among the conjugates prepared in Example 2, the conjugates of the polypeptides #1 to #14, #101 to #105, and #107 to #117 were used to perform the mouse sensitization test. A sensitizing solution was prepared by dissolving a conjugate to 0.1 mg/ml in PBS containing saponin at 10 µg/100µl, the solution was divided into fractions of 250 µl each, and the fractions were stored at -20°C. After 6-week-old mice (BALB/c) were acclimated for 1 week, conjugate solutions were administered. The sensitizing solution was administered subcutaneously at 100 µl/mouse. Administration was performed 3 or 4 times at intervals of 2 weeks. Blood sampling was performed 2 weeks after the final booster administration. After the blood sampling, serum samples were tested to measure the antibody titers by ELISA.

### [Example 3] RBD recognizing ability of mouse serum sensitized with polypeptide in RBD

Among the mouse serum samples obtained in Example 2, the serum samples obtained by sensitization with the conjugates of #1 to #14 were tested in terms of the RBD recognizing ability.

The RBD protein (No. RP01258, ABclonal) of SARS-CoV-2 was immobilized on a microwell plate at 100 mg/well, serum samples were diluted to 300-fold, 1000-fold, and 3000-fold with PBS (phosphate buffered saline), the diluted samples were dispensed to the microwell plate, and incubation was then performed for 30 minutes. Each well was washed with PBST (PBS containing 0.05% Tween 20), a solution of a horseradish peroxidase (HRP)-labeled anti-mouse IgG monoclonal antibody was added thereto, and incubation was then performed. Each well was washed with PBST, a substrate solution (a tetramethylbenzidine (TMB) solution) was added thereto, incubation was performed for a given period of time, and the reaction was then terminated with the addition of 1 N sulfuric acid. After the reaction was terminated, the absorbance at 450 nm of each well was measured using a microwell plate reader.

Figure 1 shows the absorbance of each sample. The RBD recognition abilities of mouse serum samples obtained from #3, #7, #8, and #11 were found to be high.

### [Example 4] Test of inhibition of the binding between ACE2 and RBD

Among the mouse serum samples obtained in Example 3, the samples #7 and #11 with high RBD recognizing ability were examined for the influence on the binding between ACE2 and RBD. Serum samples obtained from 5 patients (A to E) recovered from SARS-CoV-2 infection from which the consents were obtained were also examined in the same manner.

The RBD protein (No. RP01258, ABclonal) of SARS-CoV-2 was immobilized on a 96-well microwell plate, serum samples obtained from mice and serum samples obtained from patients were diluted to 100-fod, 1000-fold, and 10000-fold with PBS, the diluted samples were dispensed to the 96-well microwell plate, and incubation was then performed. In addition to the samples, a solution of a commercially available mouse anti-RBD polyclonal antibody (No. 40592-T62, Sino Biological) diluted in PBS was fractionated as a positive control, and incubation was performed in the same manner. A solution of the recombinant human ACE2 protein (No. 10108-H08H-B, Sino Biological) diluted in PBS was added to each well, and incubation was further performed. Each well was washed with PBST, a solution of the HRP-labeled anti-human ACE2 monoclonal antibody was added thereto, and incubation was then performed. Each well was washed with PBST, a substrate solution (a TMB solution) was added thereto, incubation was performed for a given period of time, and the reaction was then terminated with the addition of 1 N sulfuric acid. After the reaction was terminated, the absorbance at 450 nm of each well was measured using a microwell plate reader.

Figure 2 shows the results of the test of inhibition of the binding between ACE2 and RBD of mouse serum samples #3, #7, and #11 and a positive control sample. All the mouse serum samples were found to inhibit the binding between ACE2 and RBD. In particular, the mouse serum sample #11 diluted to 100-fold was found to exhibit the inhibitory ability equivalent to that of the positive control.

Figure 3 shows the results of the test of inhibition of the binding between ACE2 and RBD of mouse serum samples #1, #5, and #11 and serum samples obtained from patients A to E. Figure 3 (A) shows the results of comparison of inhibitory effects of mouse serum samples #1, #5, and #11. While substantially no inhibitory activity was observed in the mouse serum samples #1 and #5, inhibitory activity was observed in the mouse serum sample #11. Figure 3 (B) shows the results of comparison of inhibitory effects of the mouse serum sample #11 and the serum samples obtained from the 5 recovered patients. Except for the patient A exhibiting the highest inhibitory effect, the mouse serum sample #11 was found to exhibit the inhibitory effects on the binding between ACE2 and RBD substantially equivalent to the inhibitory effects observed in the serum samples obtained from other 4 patients.

### [Example 5] S2 domain recognizing ability of mouse serum sensitized with polypeptide at S2 domain

Among the mouse serum samples obtained in Example 3, the serum samples obtained by sensitization with the conjugates of #101 to #117 were tested as to the S2 domain recognizing ability.

The S2 protein (No. 40590-V08B, Sino Biological) of SARS-CoV-2 was immobilized on a 96-well microwell plate, serum samples were diluted to 30-fold, 100-fold, and 1000-fold with PBS, the diluted samples were dispensed to the microwell plate, and incubation was then performed. In addition to the samples, a solution of a commercially available mouse anti-S2 polyclonal antibody (No. 40590-T62, Sino Biological) diluted in PBS and PBS as a positive control were dispensed to the microwell plate, and incubation was performed in the same manner. Each well was washed with PBST, a solution of the HRP-labeled anti-mouse IgG monoclonal antibody was added thereto, and incubation was then performed. Each well was washed with PBST, a substrate solution (a TMB solution) was added thereto, incubation was performed for a given period of time, and the reaction was then terminated with the addition of 1 N sulfuric acid. After the reaction was terminated, the absorbance at 450 nm of each well was measured using a microwell plate reader.

Figure 4 shows the absorbance of each sample. In particular, the mouse serum samples #111 and #114 · #115 were found to exhibit the high S2 recognizing ability. These mouse serum samples were obtained from mice sensitized with the polypeptide of the HR2 region in the S2 domain.

### [Example 6] Test of reactivity between polypeptides in the S2 domain and serum samples obtained from recovered patients

With the use of the ELISA system using carrier antigens each presenting a polypeptide, the reactivity between the serum samples obtained from the 5 recovered patients and polypeptides in the S2 domain was examined. A 96-well microwell plate (Multisorp, NUNC) was coated with each of the antigens presenting the polypeptide #101 to #117, and the plate was blocked with a BSA/PBST solution. The serum samples obtained from the 5 patients A to E recovered from SARS-CoV-2 infection from which the consents were obtained were diluted with PBS containing BSA and then allowed to react with the polypeptide-presenting antigens. The serum samples were washed out by PBST and a solution of the HRP-labeled anti-human IgG antibody were added, and incubation was then performed for 30 minutes. After washing, a substrate solution (a tetramethylbenzidine (TMB) solution) was added thereto, and color development of the substrate was quantified at 450 nm after a given period of time.

Table 2 shows the (major) polypeptides that were recognized at the highest level in serum samples obtained from the patients A to E and the (minor) polypeptides that were recognized at the level second to the highest. The antibodies in the serum samples obtained from the 5 patients were found to strongly react with #111, #114 ▪ #115. This indicates that these sites are critical to achieve the ability to produce antibodies against SARS-CoV-2.

**[Table 3]**

| Serum from patient | **Major** | Minor |
|---|---|---|
| A | **#111** | #114 |
| B | **#114** | #105, #106 |
| C | **#111** | #105 |
| D | **#111** | #105 |
| E | **#115** | #111 |

### [Example 7] Test of sublingual administration (SLIT) of conjugate polypeptide to mice (1) Preparation of sensitizing solution for subcutaneous administration

Fractions of 17.5 µl each were obtained from solutions of four types of 1 mg/ml conjugates (#7, #11, #111, and #115) in PBS and mixed. The resulting mixture was supplemented and mixed with 3.5 µl of a solution of 100 mg/ml saponin in PBS, and the resultant was diluted to 350 µl with PBS to obtain a sensitizing solution for subcutaneous administration. A sensitizing solution for sublingual administration was stored at -20°C.

### (2) Preparation of sensitizing solution for sublingual administration

Fractions of 20 µl each were obtained from solutions of four types of 1 mg/ml conjugates (#7, #11, #111, and #115) in PBS and mixed. The resulting mixture was supplemented and mixed with 1 µl of a solution of 100 mg/ml saponin in PBS to obtain 81 µl of a sensitizing solution for sublingual administration. A sensitizing solution for sublingual administration was stored at -20°C.

### (3) Subcutaneous administration to mice

After 6-week-old mice (BALB/c) were acclimated for 1 week, conjugate solutions were administered to mice. The sensitizing solution was administered subcutaneously at 100 µl/mouse. Subcutaneous administration was performed 2 times at an interval of 2 weeks.

### (4) Sublingual administration to mice

Two weeks after the second subcutaneous administration, the sensitizing solution was administered sublingually. Mice were anesthetized using an isoflurane vaporizer, fixed in a supine position, and forced to open their mouths using forceps. The mouse tongue was lifted with the pipette chip, and 10 µl (high dose) or 3 µl (low dose) of a sensitizing solution for sublingual administration was administered dropwise sublingually. Thereafter, the mice were allowed to stand for 1 minute. Sublingual administration was performed 6 times in total at a frequency of 3 times a week, and blood samples were obtained from mice 2 days after the final administration. A group of mice subjected to high-dose sublingual administration was designated as the high-dose SLIT group and a group of mice subjected to low-dose sublingual administration was designated as the low-dose SLIT group. After the second subcutaneous administration, a group of mice subjected to the similar treatment except for sublingual administration was designated as a control group. Each group consisted of 6 mice (n = 6).

### (5) Measurement of mouse antibody titers (IgG, IgA)

The conjugate #7 and the conjugate #11 were mixed with each other at a weight ratio of 1:1 and immobilized on a 96-well microwell plate. Also, the conjugate #111 and the conjugate #115 were immobilized on different 96-well microwell plates. To the microwell plates comprising the conjugates immobilized thereon, mouse serum samples diluted in PBS were dispensed, and incubation was then performed. Each well was washed with PBST, a solution of the HRP-labeled anti-mouse IgG antibody or the HRP-labeled anti-mouse IgA antibody in PBS was added thereto, and incubation was then performed. Each well was washed with PBST, a substrate solution (a TMB solution) was added thereto, incubation was performed for a given period of time, and the reaction was then terminated with the addition of 1 N sulfuric acid. After the reaction was terminated, the absorbance at 450 nm of each well was measured using a microwell plate reader.

Figure 5 and Figure 6 each show the results of measurements of anti-RBD antibody titers of mice. Figure 5 (A) shows the reactivity of IgG in mouse serum samples of the high-dose SLIT group, the low-dose SLIT group, and the control group to the mixture of conjugates #7 and #11. Figure 5 (B) shows the reactivity of IgG in mouse serum samples of the high-dose SLIT group, the low-dose SLIT group, and the control group to the conjugate #111. Figure 5 (C) shows the reactivity of IgG in mouse serum samples of the high-dose SLIT group, the low-dose SLIT group, and the control group to the conjugate #115. Figure 6 (A) shows the reactivity of IgA in mouse serum samples of the high-dose SLIT group, the low-dose SLIT group, and the control group to the mixture of conjugates #7 and #11. Figure 6 (B) shows the reactivity of IgA in mouse serum samples of the high-dose SLIT group, the low-dose SLIT group, and the control group to the conjugate #111. Figure 6 (C) shows the reactivity of IgA in mouse serum samples of the high-dose SLIT group, the low-dose SLIT group, and the control group to the conjugate #115. In the group of mice subjected to sublingual administration of high-dose conjugates, IgG antibodies and IgA antibodies exhibiting reactivity to the conjugates significantly higher than that of the group of mice selectively subjected to subcutaneous administration were generated.

### [Example 8] Reactivity of antibodies that recognize epitopes of SARS-CoV-2 RBD molecule to antigens that present homologous epitopes of mutant RBD molecules

Mutant epitope antigens that present SARS-CoV-2 RBD epitopes each having K417N, L452R, and T478K mutations were prepared in the same manner as in Example 1.

Antigens comprising mutant RBD epitopes bound thereto were immobilized on a 96-well microwell plate at 100 ng/well. Also, a wild-type RBD epitope antigen-immobilized plate was prepared in the same manner as in Example 3. Of the anti-serum samples obtained in Example 2, the serum samples #7, #10, and #11 were diluted to 300-fold, 1500-fold, 7500-fold, 37500-fold, 188000-fold, and 940000-fold with PBS. The serum sample #7 was dispensed to a plate comprising the K417N mutant epitope antigen immobilized thereon, the serum sample #10 was dispensed to a plate comprising the L452R mutant epitope antigen immobilized thereon, and the serum sample #11 was dispensed to a plate comprising the T478K mutant epitope antigen immobilized thereon. The plates were subjected to incubation for 30 minutes, each well was washed with PBST, a solution of the HRP-labeled anti-mouse IgG monoclonal antibody was added, and incubation was then performed. Each well was washed with PBST, a substrate solution was added thereto, incubation was performed for a given period of time, and the reaction was then terminated with the addition of 1 N sulfuric acid. After the reaction was terminated, the absorbance at 450 nm of each well was measured using a microwell plate reader. The test was performed at n of 2, and the mean was determined.

Figure 7 shows the absorbances of samples. The mouse serum samples #7 and #11 were found to exhibit the sufficiently high reactivity to the mutant RBD, although such reactivity was a little lower than the reactivity to the wild-type RBD. In contrast, the mouse serum sample #10 was found to exhibit the reactivity to a homologous epitope of the mutant RBD that was substantially equivalent to the reactivity to a neutralizing epitope of the wild-type RBD. This indicates that the identified neutralizing epitope-recognizing antibody has the effects of preventing infection with SARS-CoV-2 having a mutation in the RBD protein.

### [Example 9] Effects of protecting CRFK cells from infection with SARS-CoV-2 pseudotyped lentivirus by mouse anti-serum sensitized with conjugate polypeptide

A pseudotyped lentivirus having the SARS-CoV-2 spike protein on its surface was prepared, and infected a cell expressing ACE2 on its surface to construct a system of SARS-CoV-2 pseudoinfection (see Pisil Y., et al., Pathogens, 10, 153, 2021). With the use of the pseudoinfection system, whether or not an antibody induced from the conjugate polypeptide would have neutralizing activity was evaluated in the manner described below.

### (1) Preparation of mouse antiserum samples and mixture of serum samples

A serum mixture of antiserum samples and epitope antiserum samples including the epitope recognizing antibodies prepared in Example 2 (e.g., #1, #3, #5, #7, #11, #111, #114, and #115) was prepared (the serum mixture comprises a homogeneous mixture of 2 or 3 serum samples).

### (2) Cell culture

HEK293T cells (ATCC CRL 3216) and cells derived from the feline kidney (CRFK cells, ATCC CCL-94) were cultured separately from each other in the Dulbecco's Modified Eagle Media (DMEM) supplemented with 10% (v/v) heat-inactivated fetal bovine serum, 2 mM sodium pyruvate, and 4 mM L-glutamine. Cells were collected from the culture solutions using trypsin/EDTA solutions. All the cell lines were maintained in the presence of 5% CO₂ at 37°C.

### (3) Preparation of pseudotyped lentivirus having SARS-CoV-2 spike protein

All DNA plasmids were cloned by transformation into the Escherichia coli Stbl3 cells as described in Al-Allaf, F.A., et al., Microbes Infect., Vol. 12, pp. 768-777, 2013. Pseudotyped lentivirus was constructed as described in Cronin J., et al., Curr. Gene Ther., Vol. 5, pp. 387-398, 2005. Specifically, a pseudotyped lentivirus comprising on its surface the SARS-CoV-2 spike protein derived from 1 µg/ml of the 2019-nCov_pcDNA3.1(+)-P2A-eGFP spike vector (No. MC_0101087, Molecular Cloud) and a 1 µg/ml of the HIV-1 NL4-3ΔEnvΔvpr luciferase reporter vector (pNL4-3.Luc.R-E-, No.3418, provided by NIH) (Chen B. K., et al., J. Virol., Vol. 68, pp. 654-660, 1994) were transfected into HEK293T cells (5 × 10⁵ cells/ml). Further, 200 µl of Opti-MEM (ThermoFisher) and 8 µl of a X-Treme HP transfection reagent (Sigma Aldrich) were added, and the transfected HEK293T cells were cultured in the presence of 5% CO₂ at 37°C for 2 days. The supernatant was collected and filtered through a 0.45-mm filter, so as to obtain pseudotyped lentivirus particles having the SARS-CoV-2 spike proteins on the surface.

The 2019-nCov cDNA3.1 plasmid (1 µg/ml) or the 2019-nCov CMV plasmid (1 µg/ml) was transfected into the HEK293T cells together with the pSGΔenv vector (1 µg/ml) (No. 11051, provided by NIH) to generate pseudotyped lentivirus particles comprising the SARS-CoV-2 spike proteins on the surfaces.

### (4) Preparation of ACE2-expressing cell line

CRFK cells were seeded at 2.5 × 10⁵ cells/ml in a 6-well plate containing DMEM supplemented with 10% fetal bovine serum, 2% L-glutamine, and 2% pyruvic acid. The cells were cultured for 1 day to reach 60% to 80% confluency. The pcDNA3.1+/C-(K)DYK-ACE2 plasmid (2 µg/ml) (No. MC_0101086, Molecular Cloud) was transfected into the cells using 200 µl of Opti-MEM and 8 µl of X-Treme HP transfection reagent. The transfected cells were seeded at 2.5 × 10⁵ cells/ml in a 6-well plate and incubated in the presence of 5% CO₂ at 37°C for 1 day. Thereafter, the cells were seeded in a flat-bottom 96-well microwell plate (Falcon).

### (5) Neutralization assay

The antiserum samples obtained in (1) were diluted to 18-fold, 36-fold, and 72-fold in media (the final dilution factors at the time of assay were 45-fold, 90-fold, and 180-fold). A serum mixture comprising 2 or 3 types of antiserum samples was further diluted to adjust the concentration of each serum to 1/2 or 1/3. A 720-fold dilution of commercially available IgG-positive serum from a patient recovered from severe COVID-19 (No. CoV-PosG-S, Ray Biotech) was further diluted in a medium in the same manner as described above and used as a positive control. Diluted serum solutions (180 µl each) were added to a 96-well microwell plate. A sample of pseudotyped lentivirus particles (LpVSpike(+)) having the SARS-CoV-2 spike protein on the surface was thawed and diluted to 6000 RLU (relative luciferase unit)/ml in a medium. The resultants (60 µl each) were added to the diluted serum solution (e.g., the epitope-recognizing antiserum or a serum mixture) in the 96-well microwell plate. Incubation was then performed in the presence of 5% CO₂ at 37°C for 1 hour.

ACE2-expressing CRFK cells were seeded at 2.5 × 10⁵ cells/ml in 100 µl of a medium in a 96-well microwell plate and cultured for 1 day. After incubation, diluted mouse serum solutions (150 µl each) were added to a microplate comprising the seeded CRFK cells. Incubation was then performed in the presence of 5% CO₂ at 37°C for 2 days. A well supplemented with the human anti-SARS-CoV-2 IgG antibody (No. E-AB-V1021, Elabscience) instead of the mouse serum was designated as a positive control, and a well supplemented with a medium instead of the mouse serum was designated as a negative control. A well containing neither cells or antiserum was designated as a blank. Tests were performed at n of 3. Luciferase activity of each well was measured.

Luciferase activity was measured in accordance with the method described in Ishida Y., et al., J. Gen. Virol., Vol. 97, pp. 1249-1260, 2016 and Matsuda K., et al., Virology, Vol. 399, pp. 134-143, 2010. In general, the following method was employed. A cytolytic reagent (TOYO B-Net Co., Ltd.) was added in an amount of 50 µl to each well, followed by agitation for 15 minutes. The cytolytic solution (30 µl) was collected and transferred to a 96-well microwell plate (F96 MicroWell white plate, Nunc). A luminous substrate (30 µl) was added to each well. The amount of luminescence was measured and quantified using a microplate reader (TriStar LB 941, Berthord Technologies) and the MikroWin software (Labsis Laborsysteme). The blank value was subtracted from the actually measured value, and the calculated value was designated as the measured value. The measured value of the negative control was designated to be 100%, and the relative value of the measured value was calculated. The tests were performed at n of 3.

### (6) Result

Figure 8 shows the effects of protecting ACE2-expressing CRFK cells from pseudotyped lentivirus infection by the antiserum samples obtained from mice immunized with the conjugate peptides. In Figure 8, "IgG" indicates the results concerning the positive control serum. The mouse antiserum samples immunized with the conjugate peptides #7 + #11, #7 + #111, #11 + #111, #7, and #111 and the serum mixture were found to prevent pseudovirus infection in a concentration-dependent manner. In the epitope-recognizing antibodies #1 and #5 without the RBD protein recognizing ability and a serum mixture thereof, no inhibitory activity on pseudovirus infection was observed.

All publications, patents, and patent applications cited herein are incorporated herein by reference in their entirety.

## Claims

1. A polypeptide selected from the group of polypeptides (a) to (f) below and having antibody-inducing properties:
(a) a polypeptide consisting of 17 or more continuous amino acids in a region of positions 336 to 361, 406 to 432, or 446 to 480 of SEQ ID NO: 1;
(b) a polypeptide comprising an amino acid sequence having 80% or higher sequence identity to the amino acid sequence of any polypeptide as defined in (a);
(c) a polypeptide comprising the polypeptide as defined in (a) or (b) as a partial sequence and comprising no region other than the partial sequence of SEQ ID NO: 1;
(d) a polypeptide consisting of 10 or more continuous amino acids in a region of positions 1144 to 1161 or 1174 to 1202 of SEQ ID NO: 1;
(e) a polypeptide comprising an amino acid sequence having 80% or higher sequence identity to the amino acid sequence of any polypeptide as defined in (d); and
(f) a polypeptide comprising the polypeptide as defined in (d) or (e) as a partial sequence and comprising no region other than the partial sequence of SEQ ID NO: 1.

2. The polypeptide according to claim 1, which is selected from the group of polypeptides (a) to (c) below:
(a) a polypeptide consisting of 7 or more continuous amino acids in a region of positions 336 to 361, 406 to 432, or 446 to 480 of SEQ ID NO: 1;
(b) a polypeptide comprising an amino acid sequence having 80% or higher sequence identity to the amino acid sequence of any polypeptide as defined in (a); and
(c) a polypeptide comprising the polypeptide as defined in (a) or (b) as a partial sequence and comprising no region other than the partial sequence of SEQ ID NO: 1.

3. The polypeptide according to claim 1, which is selected from the group of polypeptides (d) to (f) below:
(d) a polypeptide consisting of 10 or more continuous amino acids in a region of positions 1144 to 1161 or 1174 to 1202 of SEQ ID NO: 1;
(e) a polypeptide comprising an amino acid sequence having 80% or higher sequence identity to the amino acid sequence of any polypeptide as defined in (d); and
(f) a polypeptide comprising the polypeptide as defined in (d) or (e) as a partial sequence and comprising no region other than the partial sequence of SEQ ID NO: 1.

4. A vaccine used for treatment or prevention of SARS-CoV-2 infection comprising at least one polypeptide selected from among the polypeptides as defined in claim 1.

5. The vaccine according to claim 4, which comprises at least one polypeptide selected from among the polypeptides as defined in claim 2 and at least one polypeptide selected from among the polypeptides as defined in claim 3.

6. The vaccine according to claim 4, which comprises a conjugate of a polypeptide bound to a carrier molecule.

7. The vaccine according to claim 6, which comprises a conjugate of a plurality of polypeptides bound to a carrier molecule.

8. The vaccine according to claim 6, wherein the conjugate has a circular structure comprising polypeptides crosslinked on a carrier molecule.

9. The vaccine according to claim 4, which is a mucosal vaccine.

10. The vaccine according to claim 9, which is a sublingual vaccine.

11. An antibody-inducing composition comprising at least one polypeptide selected from among the polypeptides as defined in claim 1.

12. The polypeptide according to claim 1, which is a polypeptide consisting of 7 or more continuous amino acids in the amino acid sequence as shown in any of SEQ ID NOs: 40 to 44.
